# EUROPEAN PATENT SPECIFICATION

(11) **EP 3 553 438 B1**
(45) Date of publication and mention of the grant of the patent: **11.08.2021**
(21) Application number: 19168051.1
(22) Date of filing: 09.04.2019
(51) Int. Cl.: F26B 25/22, G05D 22/02

(54) **SYSTEM AND METHOD FOR DETECTING THE PRESENCE OF LIQUID WATER IN SANDWICH STRUCTURES**
SYSTEM UND VERFAHREN ZUR DETEKTION DES VORHANDENSEINS VON FLÜSSIGEM WASSER IN SANDWICHSTRUKTUREN
SYSTÈME ET PROCÉDÉ POUR DÉTECTER LA PRÉSENCE D'EAU LIQUIDE DANS DES STRUCTURES EN SANDWICH

(30) Priority: 10.04.2018 IT 201800004349
(43) Date of publication of application: 16.10.2019
(73) Proprietor: Leonardo S.p.A., 00195 Roma (IT)
(72) Inventor: IANNONE, Michele, 80058 Torre Annunziata (Napoli) (IT); COPPOLA, Giampiero, deceased (IT)
(74) Representative: Vanzini, Christian

(56) References cited:
- EP-A1- 2 538 207
- WO-A1-2014/057429
- TUTTLE M E: "Moisture ingression in honeycomb sandwich composites due to exposure to humidity", SAMPE CONFERENCE PROCEED; AMPE 2010 CONFERENCE AND EXHIBITION "NEW MATERIALS AND PROCESSES FOR A NEW ECONOMY", SOCIETY FOR THE ADVANCEMENT OF MATERIAL AND PROCESS ENGINEERING MARKETS AND EVOLVING TECHNOLOGIES20030511 TO 20030515LONG BEACH, CA, USA, U, 1 January 2010 (2010-01-01), pages 1-12, XP008163747, ISSN: 0891-0138

## Description

The present invention refers in general to monitoring systems of aeronautical structures.

In aeronautics, sandwich structures are often used, i.e. structures made up of two outer skins, separated by a very light core, generally made up of a honeycomb structure that separates them, allowing great flexural stiffness with a limited increase in weight. The sandwich structures may be: metallic, with skins generally made of aluminum (and sometimes titanium) and honeycomb cores made of the same material; or non-metallic, with skins made of carbon, glass or aramid fiber and polymer matrix composite and glass or aramid fiber-reinforced honeycomb cores. In both cases the skin and honeycomb are glued with adhesive films. Mark E. Tuttle discloses in "Moisture ingression in honeycomb sandwich composites due to exposure to humidity" (XP008163747) a sandwich panel with thermocouples and humidity sensors embedded within the core region.

The lightness and also the relatively competitive cost of the sandwich structures has fostered their widespread use in aeronautics. The sandwich structure with honeycomb core is however vulnerable to a problem that may limit its service life, also reducing the reliability thereof: the possibility of liquid water entering the core.

In effect, the skins are generally quite thin; the problem of the entry of liquid water may occur when the skin has breakages (even at a microscopic level) that compromise the integrity, or even, for very thin skins, porosity. The entry of water into the sandwiches for aeronautical use is also facilitated by the pressure cycles to which the sandwiches are exposed in use. In effect, in the stage of the aircraft increasing in altitude following take-off, the pressure outside the sandwich tends to decrease, and, if the sandwich is hermetically sealed, stress is created from the inside to the outside; conversely, in the stage of decreasing in altitude before landing, the pressure outside the sandwich tends to rise; this, in the presence of breakages in the skins, causes a pressure differential from the outside to the inside that, especially when passing through clouds, facilitates the entry of drops of water into the honeycomb. The phenomenon is certainly negative, also because the liquid water inside the honeycomb during flight cycles is also exposed to severe drops in temperature that cause it to solidify, with the effect of breaking the honeycomb cells through thermal expansion resulting from the solidification. The use of both metal and composite sandwich structures is therefore limited by the need for frequent checks on the presence of liquid water inside the honeycomb core. One of the methods used is infrared thermography, which is particularly sensitive to the presence of water.

One purpose of the present invention is to make available a system for detecting the presence of free liquid water in sandwich structures.

For this purpose, an object of the invention is a system for detecting liquid water in a sandwich structure, the sandwich structure comprising two outer skins and a honeycomb core that rigidly interconnects the outer skins, and wherein is obtained a plurality of cells closed at opposite ends by the two outer skins, said cells being further configured to be in fluid communication with each other, wherein the system comprises
at least one sensor placed within one of said cells, said sensor being sensitive to the presence of free liquid water within said cells, and
a processing unit configured to receive an output signal provided by the sensor and to issue a warning in the event of the presence of free liquid water within said cells.

Moreover, an object of the invention is a method for detecting liquid water in a sandwich structure, the sandwich structure comprising two outer skins and a honeycomb core that rigidly interconnects the outer skins, and wherein is obtained a plurality of cells closed at opposite ends by the two outer skins, said cells being further configured to be in fluid communication with each other,
there being provided at least one sensor placed within one of said cells, said sensor being sensitive to the presence of free liquid water within said cells,
wherein the method comprises:
receiving an output signal provided by the sensor and
issuing a warning in case of the presence of free liquid water within said cells.

Some preferred but non-limiting embodiments will now be described; making reference to the accompanying drawings, wherein:
- figure 1 is a schematic cross-section view of a portion of a sandwich panel, fitted with a monitoring system according to the invention; and
- figure 2 is a schematic plan view of the portion of the sandwich panel shown in figure 1.

With reference to the figures, a portion of a sandwich structure is represented, indicated collectively at 10.

The sandwich structure 10 comprises two outer skins respectively 11 and 13, and a honeycomb core 15 that rigidly interconnects the outer skins 11 and 13.

The outer skins 11, 13 may be made of metallic material or composite material, comprising a fiber-reinforced resin matrix.

The honeycomb core 15 may be made of metallic material or composite material, comprising a fiber-reinforced resin matrix.

The skins 11 and 13 are fixed on the two opposite sides of the honeycomb core 15, for example, by means of a structural adhesive.

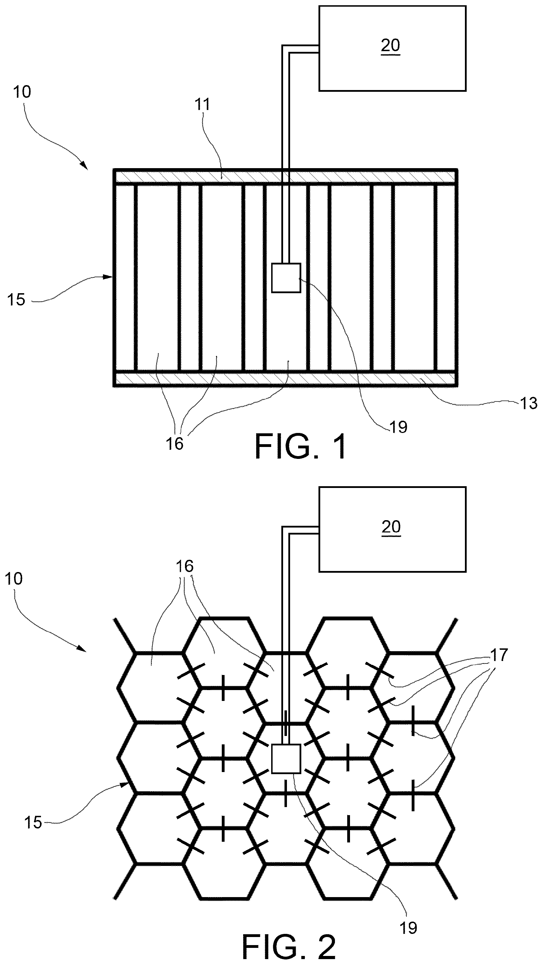

In the core 15 therefore is obtained a plurality of cells 16 that are closed on opposite ends by the two outer skins 11 and 13. The cells 16 are also configured to be in fluid communication with each other, for example, through holes made in the walls of the honeycomb material that separate the cells 16 from each other. In figure 2, the passages for the fluid are schematically represented by dashes 17 that interconnect adjacent cells 16.

At least one sensor 19 is located within one of the cells 16. Such sensor 19 is sensitive to the presence of free liquid water inside the cells 16. In particular, the sensor 19 is a humidity sensor. In the presence of liquid water, the humidity in the volume inside the core 15 is about 100%, since the core 15 is made in such a way as to allow intercommunication between the cells 16. The use of a humidity sensor, for example based on the measurement of electrical capacity, allows the presence of water to be signaled through the reading of internal humidity equal to 100% (with the measurement tolerance of the sensor).

A processing unit 20, arranged, for example, on board an aircraft on which the sandwich structure 10 is present, is configured to receive an output signal from the sensor 19. The processing unit 20 may be connected to the sensor 19 by wire or wirelessly.

Based on the output signal provided by the sensor 19, the processing unit 20 may emit a visual and/or audible warning if free liquid water is present in the cells 16 of the sandwich structure 10.

## Claims

1. A system for detecting liquid water in a sandwich structure (10), the sandwich structure (10) comprising two outer skins (11, 13) and a honeycomb core (15) that rigidly interconnects the outer skins (11, 13), and wherein is obtained a plurality of cells (16) closed at opposite ends by the two outer skins (11, 13), said cells being further configured to be in fluid communication with each other, wherein the system comprises
at least one sensor (19) placed within one of said cells, said sensor being sensitive to the presence of free liquid water within said cells, and
a processing unit (20) configured to receive an output signal provided by the sensor (19) and issue a warning in case of the presence of free liquid water within said cells.

2. System according to claim 1, wherein said sensor is a humidity sensor, and the processing unit (20) is configured to issue a warning in case of relative humidity detected by the sensor (19) equal to about 100%.

3. System according to claim 1 or 2, wherein said outer skins are made of metal material or of composite material, comprising a matrix of fiber-reinforced resin.

4. A system according to any of claims 1 to 3, wherein said honeycomb core is made of metallic material or of non-metallic material, reinforced with aramid or glass fibers.

5. A method for detecting liquid water in a sandwich structure (10), the sandwich structure (10) comprising two outer skins (11, 13) and a honeycomb core (15) that rigidly interconnects the outer skins (11, 13), and wherein is obtained a plurality of cells (16), closed at opposite ends by the two outer skins (11, 13),, said cells being further configured to be in fluid communication with each other,
there being provided at least one sensor (19) placed within one of said cells, said sensor being sensitive to the presence of free liquid water within said cells,
wherein the method comprises:
receiving an output signal provided by the sensor (19) and
issuing a warning in case of the presence of free liquid water within said cells.

6. A method according to claim 5, wherein said sensor is a humidity sensor, and wherein the method comprises:
issuing a warning in case of relative humidity detected by the sensor (19) equal to about 100%.

7. A method according to claim 5 or 6, wherein said outer skins are made of metal material or of composite material, comprising a matrix of fiber-reinforced resin.

8. A method according to any one of claims 5 to 7, wherein said honeycomb core is made of metal material or of non-metal material, reinforced with aramid or glass fibers.

## Patentansprüche

1. System zum Detektieren flüssigen Wassers in einer Sandwichstruktur (10), die zwei äußere Schichten (11, 13) und einen Wabenkern (15), der die äußeren Schichten (11, 13) starr verbindet, aufweist, und bei der eine Mehrzahl von Zellen (16), die an entgegengesetzten Enden durch die zwei äußeren Schichten (11, 13) verschlossen sind und die ferner dazu ausgebildet sind, in Fluidverbindung miteinander zu stehen, erhalten wird, wobei das System mindestens einen Sensor (19), der innerhalb einer besagter Zellen platziert ist und der für das Vorliegen freien flüssigen Wassers innerhalb besagter Zellen sensitiv ist, und
eine Verarbeitungseinheit (20), die dazu ausgebildet ist, ein Ausgangssignal, das durch den Sensor (19) zur Verfügung gestellt wird, zu empfangen und im Fall des Vorliegens freien flüssigen Wassers innerhalb besagter Zellen eine Warnung auszugeben,
aufweist.

2. System nach Anspruch 1, bei dem besagter Sensor ein Feuchtigkeitssensor ist, und die Verarbeitungseinheit (20) dazu ausgebildet ist, in einem Fall, dass eine relative Feuchtigkeit, die durch den Sensor (19) erfasst wird, gleich etwa 100% ist, eine Warnung auszugeben.

3. System nach Anspruch 1 oder 2, bei dem besagte äußere Schichten aus Metallmaterial oder aus Verbundmaterial, das eine Matrix faserverstärkten Harzes aufweist, ausgebildet sind.

4. System nach einem der Ansprüche 1 bis 3, bei dem besagter Wabenkern aus metallischem Material oder aus nichtmetallischem Material, das mit Aramid- oder Glasfasern verstärkt ist, ausgebildet ist.

5. Verfahren zum Detektieren flüssigen Wassers in einer Sandwichstruktur (10), die zwei äußere Schichten (11, 13) und einen Wabenkern (15), der die äußeren Schichten (11, 13) starr verbindet, aufweist, und bei der eine Mehrzahl von Zellen (16), die durch die äußeren Schichten (11, 13) an entgegengesetzten Enden verschlossen sind und die ferner dazu ausgebildet sind, in Fluidverbindung miteinander zu stehen, erhalten wird,
bei dem mindestens ein Sensor (19), der innerhalb einer besagter Zellen platziert ist, vorgesehen ist, der für das Vorliegen freien flüssigen Wassers innerhalb besagter Zellen sensitiv ist, wobei das Verfahren umfasst:
Empfangen eines Ausgangssignals, das durch den Sensor (19) zur Verfügung gestellt wird, und Ausgeben einer Warnung in einem Fall des Vorliegens freien flüssigen Wassers innerhalb besagter Zellen.

6. Verfahren nach Anspruch 5, bei dem besagter Sensor ein Feuchtigkeitssensor ist, und bei dem das Verfahren umfasst:
Ausgeben einer Warnung in einem Fall, dass eine relative Feuchtigkeit, die durch den Sensor (19) erfasst wird, gleich etwa 100% ist.

7. Verfahren nach Anspruch 5 oder 6, bei dem besagte äußere Schichten aus Metallmaterial oder aus Verbundmaterial, das eine Matrix faserverstärkten Harzes aufweist, ausgebildet sind.

8. Verfahren nach einem der Ansprüche 5 bis 7, bei dem besagter Wabenkern aus Metallmaterial oder aus Nichtmetallmaterial, das mit Aramid- oder Glasfasern verstärkt ist, ausgebildet ist.

## Revendications

1. Système pour détecter de l'eau liquide dans une structure en sandwich (10), la structure en sandwich (10) comprenant deux peaux extérieures (11, 13) et une âme en nid d'abeilles (15) qui interconnecte rigidement les peaux extérieures (11, 13), et dans lequel est obtenue une pluralité de cellules (16) fermées à des extrémités opposées par les deux peaux extérieures (11, 13), lesdites cellules étant en outre configurées pour être en communication fluidique les unes avec les autres, dans lequel le système comprend
au moins un capteur (19) placé dans une desdites cellules, ledit capteur étant sensible à la présence d'eau liquide libre dans lesdites cellules, et
une unité de traitement (20) configurée pour recevoir un signal de sortie prévu par le capteur (19) et émettre un avertissement en cas de présence d'eau liquide libre dans lesdites cellules.

2. Système selon la revendication 1, dans lequel ledit capteur est un capteur d'humidité, et l'unité de traitement (20) est configurée pour émettre un avertissement en cas d'humidité relative détectée par le capteur (19) égale à environ 100 %.

3. Système selon la revendication 1 ou 2, dans lequel lesdites peaux extérieures sont composées de matériau métallique ou de matériau composite, comprenant une matrice de résine renforcée par des fibres.

4. Système selon l'une quelconque des revendications 1 à 3, dans lequel ladite âme en nid d'abeilles est composée de matériau métallique ou de matériau non métallique, renforcé par des fibres d'aramide ou de verre.

5. Procédé pour détecter de l'eau liquide dans une structure en sandwich (10), la structure en sandwich (10) comprenant deux peaux extérieures (11, 13) et une âme en nid d'abeilles (15) qui interconnecte rigidement les peaux extérieures (11, 13), et dans lequel est obtenue une pluralité de cellules (16), fermées à des extrémités opposées par les deux peaux extérieures (11, 13), lesdites cellules étant en outre configurées pour être en communication fluidique les unes avec les autres,
au moins un capteur (19) placé dans une desdites cellules étant prévu, ledit capteur étant sensible à la présence d'eau liquide libre dans lesdites cellules,
dans lequel le procédé comprend :
la réception d'un signal de sortie fourni par le capteur (19) et
l'émission d'un avertissement en cas de présence d'eau liquide libre dans lesdites cellules.

6. Procédé selon la revendication 5, dans lequel ledit capteur est un capteur d'humidité, et dans lequel le procédé comprend :
l'émission d'un avertissement en cas d'humidité relative détectée par le capteur (19) égale à environ 100 %.

7. Procédé selon la revendication 5 ou 6, dans lequel lesdites peaux extérieures sont composées de matériau métallique ou de matériau composite, comprenant une matrice de résine renforcée par des fibres.

8. Procédé selon l'une quelconque des revendications 5 à 7, dans lequel ladite âme en nid d'abeilles est composée de matériau métallique ou de matériau non métallique, renforcé par des fibres d'aramide ou de verre.
